# EUROPEAN PATENT SPECIFICATION

(11) **EP 1 410 074 B1**
(45) Date of publication and mention of the grant of the patent: **17.10.2012**
(21) Application number: 02764698.3
(22) Date of filing: 15.07.2002
(51) Int. Cl.: G02B 1/04, A61L 27/16, C08F 290/04

(54) **COMPOSITIONS CAPABLE OF FORMING HYDROGELS IN THE EYE**
ZUSAMMENSTZUNGEN FÜR HYDROGELE IM AUGE
COMPOSITIONS POUVANT FORMER DES HYDROGELS DANS L'OEIL

(30) Priority: 16.07.2001 SE 0102543
(43) Date of publication of application: 21.04.2004
(73) Proprietor: AMO Groningen B.V., 9700 AX Groningen (NL)
(72) Inventor: DE GROOT, Jacqueline, Hermina, NL-9351 SR Leek (NL); HODD, Kenneth, Albert, Wrexham LL12 0PB, Wales (GB); HAITJEMA, Hendrik, Jan, NL-9321 AT Peize (NL)
(74) Representative: Holmberg, Martin Tor
(86) International application number: PCT/EP2002/007878
(87) International publication number: WO 2003/009014

(56) References cited:
- WO-A-99/47185
- WO-A1-00/22459
- WO-A1-01/89435
- US-A- 4 919 151
- US-A- 4 978 713
- US-A- 5 147 394
- US-A- 6 007 833
- US-A- 6 156 345

## Description

### Field of invention

The present invention refers to aqueous solutions of reactive polymers suitable for the production of hydrogel materials upon irradiation with blue light. The hydrogels are especially useful for production of intraocular lenses, which can be formed *in-situ* in the capsular bag in an eye having undergone surgical excision of a natural lens.

### Background of the invention

The natural lens of the human eye is a precisely formed structure of fibre cells containing about 65 percent water and 35 percent organic material, chiefly structural proteins. The proteins are responsible for the relatively high refractive index of 1.42 of the lens and are structured in such a way that there are negligible local variations in their density, resulting in a transparent lens. Aging or large stresses can change the morphology of the proteins causing a progressive loss of transparency. This is termed cataract formation and is irreversible and can eventually result in blindness.

Implantation of an intraocular lens (IOL) following cataract surgery is performed to replace the optical function of the natural lens. In order to remove the natural, cataractous lens, as well as to prepare for the introduction of the IOL, an incision is made into the eye. For many years most IOLs were made of poly(methylmethacrylate), a material with good optical characteristics and compatibility with the tissues of the eye. A disadvantage of PMMA is, however, that it is a very rigid material and the incision must be made large enough, at least 5-6 mm, for implantation of the IOL. With improved devices for less traumatic removal of the natural lens by phacoemulsification, requiring only a rather small, there was a need for lenses with deformable optics. In such small incision surgery an opening of only 3 mm, or less is required. Various silicone, acrylate and hydrogel lenses have been commercialized.

All incisions in the eye are accompanied by trauma, and so, although foldable lenses have been a great improvement, there is still a need for lenses than can be placed through an even smaller incision. A lens can be implanted through a 1.2 mm opening by injecting the lens material into the capsular bag as a fluid, followed by formation of a solid full size lens in-situ in the eye. An additional advantage of this technique is that, due to the formation of a full sized lens, complications of conventional IOL implantation namely decentration and posterior capsular opacification may be overcome. Full sized lenses show excellent centration and there is evidence that they may prevent posterior opacification (PCO). Hydrogels are a class of materials that are very interesting for an injectable lens because they have the added advantage is that their aqueous composition approximates to that of the natural lens.

Hydrogels can be made by crosslinking aqueous polymer or monomer/crosslinker solutions. Since monomers are often toxic, the use of polymers is preferred for applications in the eye. Polymers to which a reactive group is attached, for example, an acrylate group, can be polymerized in the presence of water and form a hydrogel. This is a process of crosslinking polymer or prepolymer solutions have been described before. In US Patent 5,665,840 crosslinkable water-soluble prepolymers based on copolymers of vinylalcohol and vinyllactams are claimed for contact lens application. Several other publications also disclose the production of hydrogels after crosslinking crosslinkable water-soluble (pre)polymers. The crosslinkable water-soluble (pre)polymers are designed to polymerize in a mould yield hydrogel. However, in-situ in the eye, water is present which demands certain specific requirements of the crosslinkable water-soluble prepolymers not described in any of these publications.

It is evident that the coherence of the aqueous solution during injection and prior to crosslinking is very important. This coherence prevents, or limits, the water in the eye from interfering with the solution before crosslinking. Without it, the carefully regulated hydrogel composition, required for generating an IOL of precise refractive index, may be diluted or altered and it is also needed to prevent leakage of polymer from the capsular bag before the polymer is crosslinked.

In addition, all hydrogels have an equilibrium water content, which is controlled both by the structure of the hydrogel and its crosslink density. Water-soluble polymers, when crosslinked, have a tendency to swell in water. For this invention it is important that after injecting the aqueous hydrogel solution into the eye, and crosslinking it, the amount of water that it takes up afterwards, the additional swelling, is limited, that is to say that the solution concentration of water in the injected hydrogel solution is equal to, or very close to, the equilibrium water concentration of the crosslinked hydrogel formed in the eye. Any additional swelling will decrease the refractive index of the lens and in more severe cases will cause collapsing of the capsular bag or even damage the eye.

A few publications and patents describe the design of injectable and crosslinkable water-soluble (pre)polymers and some polymerizations, which form hydrogels in-situ in the eye.

Ravi et al. reported in Polymer Preprints, 1999, 40, 630 on an injectable hydrogel material made by thermally curing poly(ethylenglycol) monoacrylates and diacrylates (PEGMA and PEGDA, respectively). They, were, however, not studied for possible lens refilling material but as potential probes to study the accommodation mechanism. The polymer content and, therefore, the refractive indices of the materials were very low (< 1.36) and the materials showed severe swelling after curing.

In the International Patent Application WO 93/02639 an injectable collagen-based intraocular hydrogel lens is described. The concentration collagen was very low which resulted in a low refractive index of the intraocular lens (1.363). It was claimed that high refractive lenses could also be made. To achieve refractive indices closer to that of the natural lens much higher collagen concentrations are needed, than those reported in the patent, and this will increase the problem of swelling, post-injection.

In the International Patent Application WO 01/08604 it was claimed that with aqueous solutions of modified linear polymers, accommodative lenses could be prepared in-situ. The water-soluble polymers were modified to low degrees, to obtain lightly crosslinked hydrogels in order to keep the elasticity modulus of the hydrogel lenses low. The moduli of the hydrogels were measured directly after crosslinking the aqueous polymer solution. The hydrogel were, however, after crosslinking not subjected to an aqueous environment. If these materials would be made in the eye in the presence of an excess natural aqueous fluid, densities they may be expected to swell significantly due to their low crosslink density. Such swelling is to the detriment of the refractive indices of the lenses they form.

In the International Patent Application WO 99/47185, a method of producing an injectable hydrogel intraocular lens is described. The hydrogel lens materials are based on macromolecular particles. However, the problems of obtaining a sufficient coherence and avoiding additional swelling are not addressed in this disclosure.

WO 01/89435 relates to expansile lenses that can form a hydrogel lens after being implanted into the eye. However, the problems of obtaining a sufficient coherence and avoiding additional swelling are not addressed in this disclosure.

WO 00/22459 relates to injectable intraocular lenses made from polysiloxanes.

US Patent No. 4,919,151 describes prepolymers that can be injected into the eye and polymerized to a lens in the presence of a photoinitiator under oxygen free conditions, but does not advice any solution to mentioned with hydrogels.

It can be concluded that the specific requirements regarding coherence and prevention of additional swelling for injectable hydrogel lenses still needs attention if a clinically acceptable surgical process is to be attained.

### Description of the invention

An object of the present invention is to provide an aqueous composition of a water soluble linear polymer that can fill the capsular bag of the eye and upon a crosslinking reaction by means of irridiation with light in the visible range can form a hydrogel intraocular lens implant of a predetermined refractive index.

It also an object of the present invention to provide an aqueous composition of a linear water soluble which has suitable coherence to avoid leaking from the capsular bag or avoiding dispersion effects in the capsular bag, while having suitably low retention time before starting the lens forming light induced crosslinking reaction.

An another object of the invention is to sufficiently crosslink the lens so that additional swelling is prevented and in this way ensure that the implanted lens permanently retains its predetermined shape and refractive value.

It is a still further object of the invention to provide hydrogel lens with no or minimal extractable polymer material.

In its most general terms the present invention pertains to an aqueous composition of a of water soluble polymers having a sufficiently high coherency that it substantially not is dispersed when being injected into an aqueous environment to undergo a crosslinking reaction into a hydrogel. The water soluble polymers can either be a water-soluble polymer bearing free acrylic, or other vinylic groups capable of facile free radical reaction with a polymeric water-soluble photoinitiator also present in the composition, or a water-soluble polymer bearing both free acrylic, or other vinylic groups capable of facile free radical reaction and photoinitiator groups. Both these polymers will be crosslinked when irradiated with light of wavelengths greater than about 305 nm induces the photoinitiator groups to form free radicals so as to form the hydrogel. Preferably, the aqueous composition shall form a clear hydrogel with a refractive index of between about 1.36 to about 1.45, a transmission of visible light of at least about 35% with no, or substantially no, absorption of water from an aqueous environment during its formation or subsequently thereafter, so it becomes dimensionally stable and substantially free from swelling after the forming crosslinking process. These features will render the aqueous compositions improved properties to form an intraocular lens, in-situ in the capsular bag of the eye, from which defect natural lens has been surgically excised.

It is an important aspect of the invention that the aqueous composition has a suitable coherence to enable a surgical procedure comprising injection into the capsular bag of the eye and crosslinking to form a lens implant replacing an excised natural crystalline lens. The coherence at zero shear stress of the aqueous composition shall be sufficiently high, so that the injected composition does not leak from the capsular bag and that the composition does not disperse when injected through a standard injection needle into an aqueous environment, such as the capsular bag of the eye. It is therefore preferred that the composition has a zero shear stress viscosity of at least 3.0 Pas, preferably 10.0 Pas, and most preferably greater than 30.0 Pas. At the same time, the coherence of the inventive aqueous composition at zero shear stress must not be too high, since this will lead to an inconveniently long relaxation time of the injected aqueous composition before it is sufficiently relaxed to assume precisely the interior contours of the capsular bag and is ready to undergo crosslinking into the lens implant. An excessively coherent aqueous composition will compromise the capacity of the capsular bag to act as a forming mould for the lens surface by exerting a backpressure, with the result of an uneven or bumpy surface of the final lens implant. The coherence of an aqueous solution is determined by the interactions between the solvated polymer molecules, and the stronger these interactions the higher the coherence. These interactions derive from some combination of the generally recognized intermolecular forces, van der Waals, polar, H-bonding, and London or dispersion forces, and for polymers an additional form of interaction, chain entanglement, is a significant contributor. Chain entanglements are physical crosslink, which occur in polymer solutions, where the polymer has a molecular weight above a context-determined threshold. The degree of chain entanglement is also concentration dependent. These various interactions combine in any given solution to determine that solution's viscosity, and so a solution's viscosity is a useful indicator of its coherence threshold.

In order to determine if the presently invented compositions exhibit a sufficient coherency, rheological tests similar to those provided in Carbohydrate Polymers, 2000, 47, 109-119 (GS Harding et al) can be followed. According to these to tests cohesive compositions of hyaluronic acid, such as Healon® 5, have a distinguishable behavior when flowing in PTFE tube that are desirable for the presently invented compositions.

In the present invention the interactions between the polymer chain is determined by the chemical nature of the modified polymer the concentration and the molecular weight. Generally, a polymer modified with a hydrophobic crosslinker, will have greater coherence for a given molecular weight and concentration than a polymer modified with a hydrophilic crosslinker, where a higher molecular weight or concentration is needed for the same coherence. It is a part of the present invention to select the polymer, its molecular weight, the functional groups for crosslinking and polymer concentration in such a way that the refractive index of the aqueous solution and the coherence are in a suitable range for an injectable solution that shall form a lens implant with a controlled and predetermined refractive value.

It is a prerequisite for the present invention that the aqueous composition shall have suitable refractive to be able to provide a lens with a refractive index similar to that of the natural crystalline lens, i.e. the composition shall have a refractive index in the range of about 1,36 to 1.45. For a given hydrophilic polymer, the refractive index of a hydrogel lens is dependent upon both the mole fraction of the polymer, and the mole fraction of water in the hydrogel and the refractive index of each. Experience has shown that, for example, when polyvinyl alcohol with a refractive index of 1.51 is used to form a hydrogel, a polymer concentration of 14 to 54 wt% is needed to obtain refractive indices of 1.36 to 1.45. For the linear, water soluble, polymers employed in the present invention, the aqueous concentration required to obtain any desired refractive index will exceed the critical concentration of about 5%. Above this concentration polymer coils start to overlap and the polymer solutions to attract water, to reduce the concentration. If the equilibrium concentration is not attained during injection swelling of the formed crosslinked gel, the lens, will result as it reverts to its equilibrium water content. According to the present invention, the tendency of swelling of the finally crosslinked lens is entirely or substantially eliminated by introducing a sufficiently high crosslink density, as is dependent by the amount of the introduced functional groups for crosslinking on the linear water soluble polymers and above exemplified with acrylate groups. The interaction parameter of the polymer and the crosslinker with water will determine the optimal crosslink density. When the polymer as well as the crosslinker have a relatively high interaction parameter with water, more crosslinks are needed to prevent additional swelling, compared to a polymer and crosslinker with a lower interaction parameter. This also counts for the interaction parameter of the polymer and crosslinker separately. If the same polymer is used, and a crosslinker with a high interaction parameter is used, the needed crosslink density is higher than in case of a crosslinker with a lower interaction parameter. With the same crosslinker, a polymer with a high interaction parameter needs more crosslinks than a polymer with a low interaction parameter. The interaction parameters χ of the polymer and crosslinker will also determine the transparency of the hydrogel. When the interaction parameter of the polymer and crosslinkers are too different, this means that the two components are not miscible which will lead to phase separation and opacification of the hydrogel. For a good transmission the interaction parameters of the crosslinker and polymer should be in the same range. In a preferred embodiment hydrophilic water soluble polymers are copolymers of vinyl alcohol and have at least one monomer unit having a lactam group, such as vinyl pyrrolidone.

Further, the copolymers have at least one monomer unit capable of acting as a functional group in the crosslinking reaction, such as an acrylate group or a derivative thereof. Advantageously these monomer units are randomly distributed on the polymer chains and present in an amount yielding between about 15 and 20 mol% of acrylate groups to obtain a sufficiently high crosslinking density. A complementing advantage of a highly crosslinked hydrogel according to the present invention, is that all the polymer chains are incorporated into the gel and so the system has no, or a minimum amount of extractable agents.

In one embodiment, the present invention relates to a mixture of two water-soluble polymers of which one is a polymer having functional groups for crosslinking attached and one is a polymer having photoinitiator groups attached. The copolymers have a general formula (A)ₘ(B)ₙ(C)ₚ(D)_{q} and (A)ₘ(B)ₙ(D)_{q}(E)ᵣ in which A can one of the following monomer: vinyl amides, vinyl lactams, acrylamide, methaerylamide, N-substitued acrylamides, N-substitued methacrylamides, vinylamine, ethyleneoxide, vinylsulfuric acids, vinylphosphonic acids, maleic acid, vinylpiperidine, vinylacrylate, methylvinylether, ethylene imine, methacrylic acid, acrylic acid, and vinylammonium salts. B is vinylalcohol or similar alcoholic group, C is a crosslinkable group attached to vinylalcohol or another alcoholic group, by its reaction with a suitable reagent such as acrylic acid chloride, methacrylic acid chloride, isocyanato acrylate, isocyanato methacrylate, epoxy acrylates, epoxy methacrylates, or itaconate or aconitate acid anhydride. D is optionally a monomer to modify the refractive index of the polymer, such as methylmethacrylate, styrene, or methyl- or benzyl-N-acetamidoacrylate, and E is a photoinitiator moiety carrying a photoinitiating group which is described per se in the International Patent Application WO 00/55214.

An alternative to this polymer mixture is a water-soluble copolymer to which both the functional group for crosslinking and the photoinitiator group are attached to the and has the following general formula (A)ₘ(B)ₙ(C)ₚ(D)_{q}(E)ᵣ where A, B, C, D, E are equally as described above.

According to a preferred embodiment, the water soluble polymer has the general formula:

(A)ₘ(B)ₙ(C)ₚ(D)_{q}

in which A, B, C and D are each are vinyl groups (-CH₂-CH-) to which a pyrrolidone group (A), a hydroxyl or acetate group (B), an acrylate group (C), and a photoinitiator group (D), respectively, is attached; and where the respective mole fractions of the different monomer units is: m=0.1-0.5; n=0.1 to 0.5; p=0.1-0.2; and q=0.0-0.1. The photoinitaitor groups and the acrylate groups are preferably randomly distributed on the polymer chains. Most advantageously, all groups A, B, C and D are randomly distributed along the chains in order to avoid zones with different hydrophilic characteristics which at worst may lead to phase separation and a compromised optical quality.

The photoinitiator groups are preferably selected so as to be able to bring about a crosslinking reaction induced by irradiation with blue light at a wavelength of about 400 to 500 nm. To accomplish this performance, the photoinitiator groups suitably comprise a phosphine oxide moiety. Typically suitable monomers (D) are 2,6-dimethylvinylbenzoyldiphenylphosphine oxide, preferably 2,6-dimethyl-4-vinylbenzoyldiphenylphosphine oxide that typically can be present in an mole fraction of amount in the range of about 0.05 to 0.25.

In a specific example of the aqueous composition the water soluble polymer can follow the formula (A)ₘ(B)ₙ(C)ₚ(D)_{q}, wherein A, B, C and D are each are vinyl groups (-CH₂-CH-) to which a pyrrolidone group (A), a hydroxyl or acetate group (B), an acrylate group (C), respectively is attached and ,where the respective mole fractions of the different monomer units are: m=0.1-0.5; n=0.1-0.5; p=0.1-0.2; and q=0. The photoinitiator is present as a separate water soluble polymer entity, which is a copolymer of 2,6-dimethyl-4-vinylbenzoyldiphenylphosphine oxide and N,N-dimethylacrylamide with 2,6-dimethyl-4-vinylbenzoyldiphenylphosphine oxide present in the water soluble photoinitiator in mole fractions in the range of 0.05 to 0.25. Alternatively, the photoinitiator is present as a separate water soluble polymer having the formula (A)ₘ(B)ₙ(D)_{q}, wherein A, B and D are each are vinyl groups (-CH₂-CH-) to which a pyrrolidone group (A), a hydroxyl or acetate group (B), and a photoinitiator group (D), respectively, is attached; and where the respective mole fractions of the different monomer units is: m=0.1 to 0.5; n=0.1 to 0.5 and q=0.05-0.25. In the photoinitaitor group (D) the phosphine oxide can be linked to the vinyl groups through a urethane band as outlined in detail in the above mentioned WO 00/55214. In this specific example of the invention the water soluble polymer to be reacted with the polymeric photoinitiator suitably is a copolymer of vinyl pyrrolidone and vinyl alcohol wherein a fraction of the hydroxyl groups modified with a functional acryl group for crosslinking. Typically about 15 to 20 mol% of the hydroxyls are modified in order to obtain the suitable crosslinking density as earlier discussed.

The aqueous compositions so far disclosed typically will find use in methods of forming a rigid and dimensionally stable hydrogel intraocular lens in situ in the eye by. The compositions will be injected with a conventional syringe into the capsular bag of the aphakic eye. Due to the suitable coherence and viscosity of the composition this can be performed without complications in the form high injection pressure, air bubble formation or material dispersion which otherwise may jeopardize patient safety or lens quality. Subsequent to injection and relaxation of the material the eye is irradiated with a sufficient amount of light exceeding about 305 nm, preferably with blue light to form the hydrogel lens

The present invention also relates a method of forming a dimensionally stable clear hydrogel that includes at first the provision of a vinyl alcohol monomer containing polymer having a refractive index of at least 1.41. A first part of the polymer is chemically modified so a sufficient amount of a first part of said vinyl alcohol monomers obtains functional acrylic groups for crosslinking. A second part of said vinyl alcohol containing polymer is chemically modified by attaching a blue light activated photoactive group comprising a phosphine oxide moiety, for example by means of an urethane bond, so as to obtain a polymeric water soluble photoinitaitor. The first and second part is mixed into an injectable aqueous composition with a sufficient polymer concentration to obtain a refractive index of about 1.36 to 1.45. Finally, the composition is injected into the eye or into another body site and irradiated with light of a wavelength exceeding about 400 nm, so as to form a hydrogel by means of crosslinking.

Besides preparation of intraocular lenses the inventive aqueous hydrogel forming compositions will find use in numerous other medical applications including tissue engineering, tissue adhesive processes. The hydrogels may also act as slow release or depot formulations for a wide variety of pharmaceuticals as for example illustrated by, but not limited to agents that inhibit epithelial cell growth on an implanted intraocular lens.

The following detailed description aims to illustrate examples of hydrogels according to present invention and should not be regarded as limiting for the scope of the invention or its applicability.

### Detailed and exemplifying part of the description

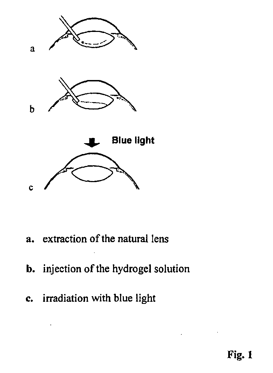

Fig. 1 shows a surgical procedure for the formation of an IOL in a pig's eye with a hydrogel forming composition according to the invention

### Examples: 1.Modification of water-soluble polymers with reactive groups

### Example 1A: Methanolysis of N-vinylpyrrolidone/vinylacetate (60/40) copolymer

The acetate groups of the copolymer were hydrolyzed by dissolving the copolymer (40.0g, 0.16 mol acetate groups, Mw 60000, Mn 20000) in methanol (200ml). A solution of NaOH (6.4 g, 0.16 mol) in water (8ml) was added to the solution. The solution was heated to 40°C for 24 h and afterwards neutralized with 2M HCl.The solution was poured into dialyzing tubes (Spectrapor 2000 MWCO 1000) and dialyzed against frequently refreshed reversed osmosis (RO) water for two days. The polymer solution was concentrated in a rotary evaporator, diluted with methanol (100ml) and precipitated in diethylether (11). The polymer, copoly(N-vinylpyrrolidone/vinylalcohol) copoly(NVP/VA), was dried in an oven, pulverized and further dried in a vacuum oven connected to an oil pump above Sicapent for at least one week. The oven was ventilated with nitrogen to prevent water absorption. The degree of hydrolysis was checked with IR (Perkin Elmer Spectrum 1).

### Example 1B: Acrylation of NVP/VA copolymer

Dried copoly(NVP/VAC) 60/40 (2.0 g, 9.5 mmol OH groups, from example 1A) was dissolved in 45 ml DMAC at 40°C under nitrogen atmosphere. 5 ml solution of 6.9 ml acryloylchloride in 43.1 ml DMAC (8.5 mmol) was added slowly to the polymer solution at room temperature. A small amount of methylhydroquinone was added and the solution was heated to 40°C overnight. The reaction mixture was protected from light by aluminum foil. The solution was neutralized with a small amount of sodium bicarbonate. The polymer was precipitated in diethylether and redissolved in 25 ml methanol to precipitate the salts. The solutions was decanted and precipitated in a 10-fold excess of diethylether. The polymer was dried in a vacuum stove. The degree of modification was determined by ¹H nmr (Varian Unity 300).

### Example 1C: Introduction of phosphineoxide groups

This reaction was performed in subdued light under an inert nitrogen atmosphere. To an oven dried three-neck flask 18.5 mol% acrylate modified copoty(NVP/VA) (2g, xmmol), as is described in example 1B was dissolved in 25 ml DMSO. To this solution, 4-isocyanato-3,5-dimethylbenzoyldiphenylphosphineoxide (0.2g, ymmol) in 10 ml DMAC was added at 40°C. After standing overnight the reaction mixture was poured into an excess of diethylether.The light yellow polymer precipitated was collected by filtration, dissolved in methanol (30ml) and reprecipitated in ether.. The polymer was dried in a vacuum oven at room temperature. A ¹H-NMR scan in DMSO-d6 of the phosphineoxide modified acrylated copoly(NVPNA) revealed that the PO substitution was 1.1 mol%.

### Example 2: The photopolymerization of a series of polyethyleneglycol diacrylates, PEGDA. (MWs in the range 250 to 4000D)

### Example 2A

A polymeric acylphosphineoxide photocrosslinker (0.050 g), for which the synthesis is described in WO 00/55214, and PEGDA (0.450 g) of which the molecular weight of the PEG part was 258, were dissolved in 0.5 g saline under dark circumstances. The solution was sucked into a syringe with an 18G needle. The solution was poured between two microscopic glass plates separated by teflon spacer (diameter 1 cm, thickness of 4 mm). The mould was illuminated with blue light (500mW/cm²) for 2 minutes. The appearance, equilibrium water content, mass loss and transmission are presented in table 1.

### Examples 2B to 20

The appearance, equilibrium water content, extractables and transmission of other gels prepared, in like manner to example 2A, from PEGDAs in a range of PEG MWs from 258 to 4000D are also collected in table 1.

### Example 3: Photopolymerization of acrylate modified copoly(NVP/VA).

### Example 3A

0.025 g PPI and 0.475 g, 2.0 mol% acrylate modified copoly(NVP/VA), were dissolved in 0.5 g saline in the dark. The solution was sucked into a syringe with an 18G needle. The polymer solution in the syringe was degassed by centrifugation at 3000 rpm. The solution was injected into a casting cell to produce a sample of a photocast gel as described in example 2A. The appearance, equilibrium water content, mass loss and transmission of this gel (3A) are presented in table 2.

### Examples 3B to 3S

The appearance, equilibrium water content, mass loss and transmission of Examples 3B to 3S, which are of gels were prepared in like manner to Example 3A, are also collected in Table 2.

**Table 1. Photocuring of polyethyleneglycoldracrylates (PEGDA) with a polymeric phosphine oxide photoinitiator (PPI)**

| **Example** | **PPI (wt% of PEGDA)** | **PEGDA+PPI (wt% in H₂O)** | **PEGDA (MW D)** | **Curing Time (s at 0.5Wcm²)** | **Appearance Of Ge**l | **EWC^{a} (wt%)** | **Extractables (wt%)** | **Transmission (% @ 480nm)** |
|---|---|---|---|---|---|---|---|---|
| **2A** | 10.0 | 50 | 258 | 120 | White | 70 ± 1 | - ^{b} | - |
| **2B** | 10.0 | 50 | 575 | 120 | Opaque | 55 ± 1 | - | - |
| **2C** | 10.0 | 50 | 700 | 120 | Slightly opaque | 56 ± 1 | - | - |
| **2D** | 10.0 | 50 | 4000 | 120 | Clear | 89 ± 1 | - | - |
| **2E** | 1.0 | 50 | 700 | 120 | Clear | 54 ± 1 | - | - |
| **2F** | 2.5 | 50 | 700 | 120 | Clear | 54 ± 1 | - | - |
| **2G** | 5.0 | 50 | 700 | 120 | Clear | 54 ± 1 | - | - |
| **2H** | 5.0 | 50 | 700 | 10 s | Clear | 54 ± 1 | 1.7 ± 0.5 | 89 ± 1 |
| **2I** | 5.0 | 50 | 700 | 30 s | Clear | 54 ± 1 | 1.4 ± 0.5 | 91 ± 1 |
| **2J** | 5.0 | 50 | 700 | 120 s | Clear | 54 ± 1 | 0.9 ± 0.5 | 84 ± 1 |
| **2K** | 5.0 | 50 | 700 | 150 s | Clear | 54 ± 1 | 1.4 ± 0.5 | 87 ± 1 |
| **2L** | 0.5 | 50 | 700 | 30 s (at 2.0Wcm²) | Clear | 57 ± 1 | 1.5 ± 0.5 | 76 ± 1 |
| **2M** | 1.0 | 50 | 700 | 30 s (at 2.0Wcm²) | Clear | 54 ± 1 | 0.7 ± 0.5 | 90 ± 1 |
| **2N** | 2.5 | 50 | 700 | 30 s (at 2.0Wcm²) | Clear | 54 ± 1 | 1.2 ± 0.5 | 90 ± 1 |
| **2O** | 5.0 | 50 | 700 | 30 s (at 2.0Wcm²) | Clear | 54 ± 1 | 1.0 ± 0.5 | 91 ± 1 |

| | | | | | | | | |
|---|---|---|---|---|---|---|---|---|
| ^{a} Equilibrium water content, ^{b} not measured | | | | | | | | |

**Table 2. Cast gels prepared by photopolymerisation of acrylated copolyNVP/VA & PPI**

| **Example #** | **PPI (w% of NVPNA)** | **PPI+NVP/VA (wt% in H₂O)** | **Acrylate (mole% of NVP/VA)** | **Curing time (min @ 0.5W)** | **Appearance** | **EWC*^{a}* (wt%)** | **MassLoss (wt%)** | **Transmission (% @ 480mm)** |
|---|---|---|---|---|---|---|---|---|
| **3A** | 5.0 | 50 | 2.0 ± 0.5 | 2 | White | 81 ± 1 | - *^{b}* | - |
| **3B** | 5.0 | 50 | 4.5 ± 0.5 | 2 | Opaque | 78 ± 1 | - | - |
| **3C** | 5.0 | 50 | 8.0 ± 0.5 | 2 | Opaque | 56 ± 1 | - | - |
| **3D** | 5.0 | 50 | 12.0 ± 0.5 | 2 | Slightly opaque | 52 ± 1 | - | - |
| **3E** | 5.0 | 50 | 15.0 ± 0.5 | 2 | Translucent | 52 ± 1 | - | - |
| **3F** | 5.0 | 50 | 16.2 ± 0.5 | 2 | Yellow clear | 52 ± 1 | 0.0 | 25 ± 1 |
| **3G** | 5.0 | 50 | 17.7 ± 0.5 | 2 | Yellow clear | 52 ± 1 | 0.0 | 28 ± 1 |
| **3H** | 5.0 | 50 | 19.9 ± 0.5 | 2 | Translucent | 52 ± 1 | - | - |
| **3I** | 5.0 | 50 | 18.5 ± 0.5 | 0.5 | Fluid | - | - | - |
| **3J** | 5.0 | 50 | 18.5 ± 0.5 | 1 | Fluid | - | - | - |
| **3K** | 5.0 | 50 | 18.5 ± 0.5 | 1.5 | White pieces | - | - | - |
| **3L** | 5.0 | 50 | 18.5 ± 0.5 | 2 | Yellow clear | 52 ± 1 | 0.0 ± 0.5 | 33 ± 1 |
| **3M** | 5.0 | 50 | 18.5 ± 0.5 | 3 | Yellow clear | 52 ± 1 | 0.0 ± 0.5 | 35 ± 1 |
| **3N** | 5.0 | 50 | 18.5 ± 0.5 | 4 | Yellow clear | 51 ± 1 | 0.0 ± 0.5 | 37 ± 1 |
| **3O** | 5.0 | 50 | 18.5 ± 0.5 | 2(@ 2Wcm²) | Yellow clear | 51 ± 1 | 0.0 ± 0.5 | 40 ± 1 |
| **3P** | 1.0 | 50 | 15.0 ± 0.5 | 2 | Fluid | - | - | - |
| **3Q** | 2.5 | 50 | 15.0 ± 0.5 | 2 | Opaque | - | - | - |
| **3R** | 5.0 | 50 | 15.0 ± 0.5 | 2 | Yellow clear | - | - | - |
| **3S** | 10 | 50 | 15.0 ± 0.5 | 2 | Opaque | - | - | - |

| | | | | | | | | |
|---|---|---|---|---|---|---|---|---|
| *^{a}* Equilibrium water content, *^{b}* not measured | | | | | | | | |

### Example 4

Method described in example 3A was repeated with 0.5 g polymer described in example 1B. The hydrogel was comparable to hydrogel described in example 3L.

### Example 5: The in-vitro photopolymerization of hydrogels in the capsular bags of pigs' eyes.

### Example 5A

The surgical procedure of the lens formation in the capsular bag of an extracted pig's eye is shown in Figure 1. After removal of the natural lens, a solution of photocurable hydrogel with a viscosity at zero shear rate of 0.15Pas, as is described in example 3L, was injected into the capsular bag and cured by irradiating with blue light at 2 W/cm².

### Examples 5B to 5G

Following the method described for example 5A lenses were prepared in pigs' eyes from hydrogel solutions with viscosities at zero shear rate in the range 0.80 to 200 Pas. The shapes of the lenses formed are presented in Table 3.

**Table 3 Viscosities at zero shear rate of solutions used to form lenses in pigs' eyes and the resulting lens shapes.**

| **Example #** | **η (Pas)** | **Lens shape^{a}** |
|---|---|---|
| 5A | 0.15 | - |
| 5B | 0.80 | - |
| 5C | 3.0 | ± |
| 5D | 12 | + |
| 5E | 30 | + |
| 5F | 80 | + |
| 5G | 200 | + |

| | | |
|---|---|---|
| ^{a} Incompletely formed "-" Partially formed "±" Well formed "+" | | |

## Claims

1. An aqueous composition of water soluble polymers having a coherency effective to prevent dispersion when the composition is injected into an aqueous environment to undergo a crosslinking reaction into a hydrogel and having a zero shear stress viscosity of at least 3.0 Pas, wherein the water soluble polymers comprises
(i) a water-soluble polymer bearing free acrylic, or other vinylic groups capable of facile free radical reaction with a polymeric water-soluble photoinitiator also present in the composition, or
(ii) a water-soluble polymer bearing both free acrylic, or other vinylic groups capable of facile free radical reaction and photoinitiator, wherein
the water soluble polymer is a linear polymer capable of providing the resulting hydrogel with (i) a refractive index of between 1.36 and 1.45; (ii) a transmission of visible light of at least 35%; and (iii) no or substantially no absorption of water from an aqueous environment during its formation or subsequently, and so is dimensionally stable and substantially free from swelling, and
wherein the water soluble polymer is present in a concentration of 14 to 54 wt % and the water soluble polymer is capable of being crosslinked when irradiated with light of wavelengths greater than about 305 nm to form the hydrogel.

2. An aqueous compositions according to claim 1, wherein said hydrophilic water soluble polymers are copolymers of vinyl alcohol.

3. A composition according to claim 2, wherein said copolymers have at least one monomer unit having a lactam group.

4. A composition according to claim 3, wherein said monomer unit is vinyl pyrrolidone

5. A composition according to any of claims 2 to 4, wherein said copolymers further have at least one monomer unit capable of acting as a functional group in the crosslinking reaction.

6. A compositions according to claim 5, wherein said monomer unit comprises an acrylate group or a derivative thereof.

7. A composition according to claim 5 wherein said monomer unit is randomly distributed on the polymer chains.

8. A composition according to claim 6, wherein said polymer comprises between 15 and 20 mol% of said acrylate groups.

9. A composition according to claim 1 wherein the water soluble polymers have the general formulas (A)ₘ(B)ₙ(C)ₚ(D)_{q} or (A)ₘ(B)ₙ(D)_{q}(E)ᵣ in which A can one of the following monomer: vinyl amides, vinyl lactams, acrylamide, methacrylamide, N-substitued acrylamides, N-substitued methacrylamides, vinylamine, ethyleneoxide, vinylsulfuric acids, vinylphosphonic acids, maleic acid, vinylpiperidine, vinylacrylate, methylvinylether, ethylene imine, methacrylic acid, acrylic acid, and vinylammonium salts, B is vinylalcohol or similar alcoholic group, C is a crosslinkable group attached to vinylalcohol or another alcoholic group, by its reaction with a suitable reagent such as acrylic acid chloride, methacrylic acid chloride, isocyanato acrylate, isocyanato methacrylate, epoxy acrylates, epoxy methacrylates, or itaconate or aconitate acid anhydride, D is optionally a monomer to modify the refractive index of the polymer, such as methylmethacrylate, styrene, or methyl- or benzyl-N-acetamidoacrylate, and E is a photoinitiator moiety carrying a photoinitiating group.

10. A composition according to any of claims 2 to 9, wherein said water soluble polymers have the general formula:
(A)ₘ(B)ₙ(C)ₚ(D)_{q}
in which A, B, C and D are each are vinyl groups (-CH₂-CH-) to which a pyrrolidone group (A), a hydroxyl or acetate group (B), an acrylate group (C), and a photoinitiator group (D), respectively, is attached; and where the respective mole fractions of the different monomer units is: m=0.1-0.5; n=0.1 to 0.5; p=0.1 to 0.2; and q=0.0 to 0.1.

11. A composition according to any of claim 9 or 10, wherein said photoinitiator group is randomly distributed on the polymer chains.

12. A composition according to any of claims 1 to 11 **characterized in that** the crosslinking reaction is performed with blue light at a wavelength of 400 to 500 nm.

13. A composition according to claim 12, wherein the photoinitiator group comprises a phosphine oxide moiety.

14. A composition according to claim 13, wherein the monomer unit bearing the phosphine oxide moiety is 2,6-dimethylvinylbenzoyldiphenylphosphine oxide, where the preferred monomer unit bearing the phosphine oxide moiety is 2,6-dimethyl-4-vinylbenzoyldiphenylphosphine oxide.

15. A composition according to claim 1 comprising a water soluble polymer according to any of claims 2 to 9 free of monomers bearing photoinitiator groups and a water soluble polymeric photoinitiator bearing photoinitiator groups that is a copolymer of 2,6-dimethyl-4-vinylbenzoyldiphenylphosphine oxide and N,N-dimethylacrylamide.

16. A composition according to claim 15, wherein the 2,6-dimethyl-4-vinylbenzoyldiphenylphosphine oxide is present in the water soluble photoinitiator in mole fractions in the range of 0.05 to 0.25.

17. A composition according to claim 1 comprising a water soluble polymer according to any of claims 4 to 12 free of monomers bearing photoinitiator groups and a water soluble polymeric photoinitiator having the formula:
(A)ₘ(B)ₙ(D)_{q}
in which A, B and D are each are vinyl groups (-CH₂-CH-) to which a pyrrolidone group (A), a hydroxyl or acetate group (B), and a photoinitiator group (D), respectively, is attached; and where the respective mole fractions of the different monomer units is: m=0.1 to 0.5; n=0.1 to 0.5 and q=0.05 to 0.25.

18. A composition according to claim 17, wherein the photoinitiator group comprises a phosphine oxide moiety linked to the main polymer chain by a urethane bond.

19. A composition according to claim 18, wherein the water soluble polymer free of monomers bearing photoinitiator groups is a copolymer of vinylpyrrolidone and vinyl alcohol having a fraction of its hydroxyl group modified with a functional acryl group for crosslinking.

20. A composition according to claim 19, wherein the water soluble polymeric photoinitiator is a copolymer of vinylpyrrolidone and vinyl alcohol having a phosphine oxide moiety attached thereto by means of a urethane bond.

21. An intraocular lens formed from any of the compositions according claims 1-20.

22. A hydrogel formed from any of the compositions according to claims 1-20, having water equilibrium content less than about 50% wt, a transmission of light at 480 nm in the range of 35 to 45% and being free from additional swelling after crosslinking.

## Patentansprüche

1. Wässrige Zusammensetzung aus wasserlöslichen Polymeren, die eine Kohärenz aufweisen, die eine Dispersion wirksam verhindert, wenn die Zusammensetzung in eine wässrige Umgebung injiziert wird, um eine Vernetzungsreaktion zu einem Hydrogel einzugehen, und die bei Nullscherspannung eine Viskosität von mindestens 3,0 Pas aufweist, wobei die wasserlöslichen Polymere
(i) ein wasserlöslichesPolymer, das freie Acrylgruppenoder andere Vinylgruppen trägt, die zum Ermöglichen einer Reaktion von freien Radikalen mit einem polymeren wasserlöslichen Photoinitiator, der ebenfalls in der Zusammensetzung vorhanden ist, in der Lage sind, oder
(ii) ein wasserlöslichesPolymer, das sowohl freie Acrylgruppen als auch andere Vinylgruppen trägt, die zum Ermöglichen einer Reaktion von freien Radikalen in der Lage sind, und einen Photoinitiator
aufweisen, wobei
es sich bei dem wasserlöslichen Polymer um ein lineares Polymer handelt, das in der Lage ist, das resultierende Hydrogel mit (i) einem Brechungsindex zwischen 1,36 und 1,45, (ii) einer Transmission von sichtbarem Licht von mindestens 35 % und (iii) keiner oder im Wesentlichen keiner Absorption von Wasser aus einer wässrigen Umgebung während seiner Bildung oder im Anschluss daran zu versehen, und es deshalb dimensional stabil und frei von einer Volumenzunahme ist, und
wobei das wasserlösliche Polymer in einer Konzentration von 14 Gew.-% bis 54 Gew.-% vorhanden ist und das wasserlösliche Polymer vernetzt werden kann, wenn es mit Licht einer Wellenlänge von mehr als 305 nm bestrahlt wird, um das Hydrogel zu bilden.

2. Wässrige Zusammensetzungen nach Anspruch 1, wobei es sich bei den hydrophilen wasserlöslichen Polymeren um Copolymere von Vinylalkohol handelt.

3. Zusammensetzung nach Anspruch 2, wobei die Copolymere mindestens eine Monomereinheit mit einer Lactamgruppe aufweisen.

4. Zusammensetzung nach Anspruch 3, wobei es sich bei der Monomereinheit um Vinylpyrrolidon handelt.

5. Zusammensetzung nach einem der Ansprüche 2 bis 4, wobei die Copolymere des Weiteren mindestens eine Monomereinheit aufweisen, die zum Wirken als funktionelle Gruppe in der Vernetzungsreaktion in der Lage ist.

6. Zusammensetzung nach Anspruch 5, wobei die Monomereinheit eine Acrylatgruppe oder ein Derivat davon aufweist.

7. Zusammensetzung nach Anspruch 5, wobei die Monomereinheit zufällig auf den Polymerketten verteilt ist.

8. Zusammensetzung nach Anspruch 6, wobei das Polymer zwischen 15 Mol.-% und 20 Mol.-% der Acrylatgruppen aufweist.

9. Zusammensetzung nach Anspruch 1, wobei die wasserlöslichen Polymere die allgemeinen Formeln (A)ₘ(B)ₙ(C)ₚ(D)_{q} oder (A)ₘ(B)ₙ(D)_{q}(E)ᵣ aufweisen, wobei A eines der folgenden Monomere sein kann: Vinylamide, Vinyllactame, Acrylamid, Methacrylamid, N-substituierte Acrylamide, N-substituierte Methacrylamide, Vinylamin, Ethylenoxid, Vinylschwefelsäuren, Vinylphosphonsäuren, Maleinsäure, Vinylpiperidin, Vinylacrylat, Methylvinylether, Ethylenimin, Methacrylsäure, Acrylsäure und Vinylammoniumsalze, B Vinylalkohol oder eine ähnliche alkoholische Gruppe ist, C eine Vernetzungsgruppe ist, die durch seine Reaktion mit einem geeigneten Reagens wie etwa Acrylsäurechlorid, Methacrylsäurechlorid, Isocyanatoacrylat, Isocyanatomethacrylat, Epoxyacrylaten, Epoxymethacrylaten oder Itaconat oder Aconitatsäureanhydrid an Vinylalkohol oder einer anderen alkoholischen Gruppe angebracht ist, D wahlweise ein Monomer ist, um den Brechungsindex des Polymers zu verändern, beispielsweise Methylmethacrylat, Styrol oder Methyl- oder Benzyl-N-acetamidoacrylat, und E eine Photoinitiator-Einheit ist, welche eine photoinitiierende Gruppe trägt.

10. Zusammensetzung nach einem der Ansprüche 2 bis 9, wobei die wasserlöslichen Polymere die allgemeine Formel:
(A)m(B)n(C)p(D)q
aufweisen, wobei es sich bei A, B, C und D jeweils um Vinylgruppen (-CH₂-CH-) handelt, an die eine Pyrrolidongruppe (A), eine Hydroxyl- oder Acetatgruppe (B), eine Acrylatgruppe (C) bzw. eine Photoinitiatorgruppe (D) angebracht sind, und wobei die jeweiligen Molanteile der verschiedenen Monomereinheiten m=0,1-0,5, n=0,1 bis 0,5, p=0,1 bis 0,2 und q=0,0 bis 0,1 betragen.

11. Zusammensetzung nach einem der Ansprüche 9 oder 10, wobei die Photoinitiatorgruppe auf den Polymerketten zufällig verteilt ist.

12. Zusammensetzung nach einem der Ansprüche 1 bis 11, **dadurch gekennzeichnet, dass** die Vernetzungsreaktion mit blauem Licht einer Wellenlänge von 400 nm bis 500 nm durchgeführt wird.

13. Zusammensetzung nach Anspruch 12, wobei die Photoinitiatorgruppe eine Phosphinoxid-Einheit aufweist.

14. Zusammensetzung nach Anspruch 13, wobei es sich bei der Monomereinheit, welche die Phosphinoxid-Einheit trägt, um 2,6-Dimethylvinylbenzoyldiphenylphosphinoxid handelt, wobei es sich bei der bevorzugten Monomereinheit, welche die Phosphinoxid-Einheit trägt, um 2,6-Dimethyl-4-vinylbenzoyldiphenylphosphinoxid handelt.

15. Zusammensetzung nach Anspruch 1, die ein wasserlösliches Polymer nach einem der Ansprüche 2 bis 9, das frei von Monomeren ist, welche Photoinitiatorgruppen tragen, und einen wasserlöslichen polymeren Photoinitiator aufweist, der Photoinitiatorgruppen trägt und bei dem es sich um ein Copolymer von 2,6-Dimethyl-4-vinylbenzoyldiphenylphosphinoxid und N,N-Dimethylacrylamid handelt.

16. Zusammensetzung nach Anspruch 15, wobei das 2,6-Dimethyl-4-vinylbenzoyldiphenylphosphinoxid in dem wasserlöslichen Photoinitoator in Molanteilen im Bereich von 0,05 bis 0,25 vorhanden ist.

17. Zusammensetzung nach Anspruch 1, die ein wasserlösliches Polymer nach einem der Ansprüche 4 bis 12, das frei von Monomeren ist, welche Photoinitiatorgruppen tragen, und einen wasserlöslichen polymeren Photoinitiator mit der Formel:
(A)ₘ(B)ₙ(D)_{q}
aufweist, wobei es sich bei A, B und D jeweils um Vinylgruppen (-CH₂-CH-) handelt, an die eine Pyrrolidongruppe (A), eine Hydroxyl- oder Acetatgruppe (B), bzw. eine Photoinitiatorgruppe (D) angebracht sind, und wobei die jeweiligen Molanteile der verschiedenen Monomereinheiten m=0,1 bis 0,5, n=0,1 bis 0,5 und q=0,05 bis 0,25 betragen.

18. Zusammensetzung nach Anspruch 17, wobei die Photoinitiatorgruppe eine durch eine Urethanbindung mit der Polymerhauptkette verknüpfte Phosphinoxid-Einheit aufweist.

19. Zusammensetzung nach Anspruch 18, wobei es sich bei dem wasserlöslichen Polymer, das frei von Monomeren ist, welche Photoinitiatorgruppen tragen, um ein Copolymer von Vinylpyrrolidon und Vinylalkohol handelt, wobei ein Anteil seiner Hydroxylgruppe zum Vernetzen mit einer funktionellen Acrylgruppe modifiziert ist.

20. Zusammensetzung nach Anspruch 19, wobei es sich bei dem wasserlöslichen polymeren Photoinitiator um ein Copolymer von Vinylpyrrolidon und Vinylalkohol handelt, an den über eine Urethanbindung eine Phosphinoxideinheit angebracht ist.

21. Intraokuläre Linse, die aus einer der Zusammensetzungen nach Anspruch 1-20 gebildet ist.

22. Hydrogel, das aus einer der Zusammensetzungen nach Anspruch 1-20 gebildet ist, einen Gleichgewichtswassergehalt von weniger als 50 Gew.-% und eine Lichttransmission bei 480 nm im Bereich von 35 % bis 45 % aufweist und nach dem Vernetzen frei von einer zusätzlichen Volumenzunahme ist.

## Revendications

1. Composition aqueuse de polymères hydrosolubles ayant une cohésion efficace pour empêcher la dispersion lorsque la composition est injectée dans un environnement aqueux pour y subir une réaction de réticulation donnant un hydrogel et ayant une viscosité à cisaillement nul au moins égale à 3,0 Pats, lesdits polymères hydrosolubles comprenant :
i) un polymère hydrosoluble portant des groupes acryliques ou d'autres groupes vinyliques libres capables de réagir facilement avec des radicaux libres en présence d'un photoamorceur polymère hydrosoluble également contenu dans la composition, ou
ii) un polymère hydrosoluble portant à la fois des groupes acryliques ou d'autres groupes vinyliques libres capables de réagir facilement avec des radicaux libres et un photoamorceur, dans laquelle
le polymère hydrosoluble est un polymère linéaire capable de conférer à l'hydrogel résultant (i) un indice de réfraction dans la plage de 1,36 à 1,45 ; (ii) une transmission de la lumière visible au moins égale à 35% ; et (iii) une absorption d'eau à partir d'un environnement aqueux nulle ou pratiquement nulle au cours de sa formation ou par la suite, de façon qu'il soit dimensionnellement stable et exempt de gonflement, et
dans laquelle le polymère hydrosoluble est présent à une concentration dans la plage de 14 à 54% en poids et le polymère hydrosoluble est capable de réticuler lorsqu'il est exposé à de la lumière de longueur d'onde supérieure à 305 nm pour former l'hydrogel.

2. Composition aqueuse selon la revendication 1, dans laquelle lesdits polymères hydrosolubles hydrophiles sont des copolymères d'alcool vinylique.

3. Composition selon la revendication 2, dans laquelle lesdits copolymères possèdent au moins un motif monomère contenant un groupe lactame.

4. Composition selon la revendication 3, dans laquelle ledit motif monomère est la vinylpyrrolidone.

5. Composition selon l'une quelconque des revendications 2 à 4, dans laquelle lesdits copolymères comprennent en plus au moins un motif monomère capable d'agir comme un groupe fonctionnel dans la réaction de réticulation.

6. Composition selon la revendication 5, dans laquelle ledit motif monomère comprend un groupe acrylate ou un dérivé de celui-ci.

7. Composition selon la revendication 5, dans laquelle ledit motif monomère est distribué de manière aléatoire sur les chaînes polymères.

8. Composition selon la revendication 6, dans laquelle ledit polymère comprend de 15 à 20% en moles desdits groupes acrylate.

9. Composition selon la revendication 1, dans laquelle les polymères hydrosolubles ont les formules générales (A)ₘ(B)ₙ(C)ₚ(D)_{q} ou (A)ₘ(B)ₙ(D)_{q}(E)ᵣ, où A peut être un des monomères suivants : amides vinyliques, lactames vinyliques, acrylam ide, méthacrylam ide, acrylam ides N-substitués, méthacrylam ides N-substitués, vinylamine, oxyde d'éthylène, acides vinylsulfuriques, acides vinylphosphoniques, acide maléique, vinylpipéridine, acrylate de vinyle, méthylvinyléther, éthylène imine, acide méthacrylique, acide acrylique et sels de vinylammonium, B est l'alcool vinylique ou un groupe alcoolique similaire, C est un groupe réticulable rattaché à l'alcool vinylique ou un autre groupe alcoolique en le faisant réagir avec un réactif adéquat tel que le chlorure d'acide acrylique, le chlorure d'acide méthacrylique, l'isocyanatoacrylate, l'isocyanatométhacrylate, des époxyacrylates, des époxyméthacrylates ou l'anhydride itaconique ou aconitique, D est facultativement un monomère servant à modifier l'indice de réfraction du polymère, par exemple le méthacrylate de méthyle, le styrène ou le N-acétamido-acrylate de méthyle ou benzyle, et E est un fragment photoamorceur portant un groupe photoamorceur.

10. Composition selon l'une quelconque des revendications 2 à 9, dans laquelle lesdits polymères hydrosolubles ont la formule générale (A)ₘ(B)ₙ(C)ₚ(D)_{q}, où A, B, C et D sont chacun des groupes vinyle (-CH₂-CH-) auxquels un groupe pyrrolidone (A), un groupe hydroxyle ou acétate (B), un groupe acrylate (C) et un groupe photoamorceur (D), respectivement, est rattaché ; et où les fractions molaires respectives des différents motifs monomères ont les valeurs suivantes : m=0,1 à0,5; n=0,1 à0,5; p=0,1 à0,2; et q = 0,0 à 0,1.

11. Composition selon l'une quelconque des revendications 9 ou 10, dans laquelle ledit groupe photo-amorceur est distribué de manière aléatoire sur les chaînes polymères.

12. Composition selon l'une quelconque des revendications 1 à 11, **caractérisée en ce que** la réaction de réticulation se fait en utilisant de la lumière bleue à une longueur d'onde de 400 à 500 nm.

13. Composition selon la revendication 12, dans laquelle le groupe photo-amorceur comprend un fragment oxyde de phosphine.

14. Composition selon la revendication 13, dans laquelle le motif monomère portant le fragment oxyde de phosphine est l'oxyde de 2,6-diméthylvinylbenzoyl-diphénylphosphine, le motif monomère portant le fragment oxyde de phosphine préféré étant l'oxyde de 2,6-diméthyl-4-vinylbenzoyldiphénylphosphine.

15. Composition selon la revendication 1, comprenant un polymère hydrosoluble selon l'une quelconque des revendications 2 à 9 exempt de monomères portant des groupes photoamorceurs et un photoamorceur polymère hydrosoluble portant des groupes photoamorceurs qui est un copolymère d'oxyde de 2,6-diméthyl-4-vinylbenzoyldiphénylphosphine et de N,N-diméthylacrylamide.

16. Composition selon la revendication 15, dans laquelle l'oxyde de 2,6-diméthyl-4-vinylbenzoyldiphénylphosphine est présent dans le photoamorceur hydrosoluble à des fractions molaires dans la plage de 0,05 à 0,25.

17. Composition selon la revendication 1, comprenant un polymère hydrosoluble selon l'une quelconque des revendications 4 à 12 exempt de monomères portant des groupes photoamorceurs et un photoamorceur polymère hydrosoluble de formule (A)ₘ(B)ₙ(D)_{q}, où A, B et D sont chacun des groupes vinyle (-CH₂-CH-) auxquels un groupe pyrrolidone (A), un groupe hydroxyle ou acétate (B) et un groupe photoamorceur (D), respectivement, est rattaché ; et où les fractions molaires respectives des différents motifs monomères ont les valeurs suivantes : m = 0,1 à 0,5 ; n = 0,1 à 0,5 ; et q = 0,05 à 0,25.

18. Composition selon la revendication 17, dans laquelle le groupe photo-amorceur comprend un fragment oxyde de phosphine rattaché à la chaîne principale du polymère par le biais d'un pont uréthane.

19. Composition selon la revendication 18, dans laquelle le polymère hydrosoluble exempt de monomères portant des groupes photoamorceurs est un copolymère de vinylpyrrolidone et d'alcool vinylique dont une fraction des groupes hydroxyle est modifiée par un groupe acryle fonctionnel pour la réticulation.

20. Composition selon la revendication 19, dans laquelle le photoamorceur polymère hydrosoluble est un copolymère de vinylpyrrolidone et d'alcool vinylique auquel un fragment oxyde de phosphine est rattaché par le biais d'un pont uréthane.

21. Lentille intraoculaire formée à partir de l'une quelconque des compositions selon les revendications 1 à 20.

22. Hydrogel formé à partir de l'une quelconque des compositions selon les revendications 1 à 20, ayant une teneur en eau à l'équilibre inférieure à 50% en poids et une transmission de la lumière à 480 nm dans la plage de 35 à 45%, et exempt de gonflement additionnel après la réticulation.
